# EUROPEAN PATENT SPECIFICATION

(11) **EP 3 231 361 B1**
(45) Date of publication and mention of the grant of the patent: **12.03.2025**
(21) Application number: 15876429.0
(22) Date of filing: 05.01.2015
(51) Int. Cl.: A61B 5/02, A61B 5/00, A61B 5/01, A61B 5/021, A61B 5/024, A61B 5/0285, A61B 5/145

(54) **DETECTION METHOD FOR WEARABLE DEVICE, AND WEARABLE DEVICE**
DETEKTIONSVERFAHREN FÜR WEARABLE-VORRICHTUNG SOWIE WEARABLE-VORRICHTUNG
PROCÉDÉ DE DÉTECTION D'UN DISPOSITIF VESTIMENTAIRE ET DISPOSITIF VESTIMENTAIRE

(43) Date of publication of application: 18.10.2017
(73) Proprietor: Huawei Technologies Co., Ltd., Longgang District Shenzhen, Guangdong 518129 (CN)
(72) Inventor: LI, Liang, Shenzhen Guangdong 518129 (CN); QIAN, Zexu, Shenzhen Guangdong 518129 (CN)
(74) Representative: Maiwald GmbH
(86) International application number: PCT/CN2015/070093
(87) International publication number: WO 2016/109918

(56) References cited:
- EP-A1- 2 549 249
- CN-A- 102 525 436
- CN-A- 103 257 873
- CN-A- 103 536 279
- CN-A- 103 943 106
- CN-U- 202 842 494
- CN-U- 203 136 125
- US-A1- 2006 253 010
- US-A1- 2008 045 806
- US-A1- 2012 215 115
- US-A1- 2014 275 852

## Description

### TECHNICAL FIELD

The present invention relates to the communications field, and in particular, to a method for detecting a wearable device, and the wearable device.

### BACKGROUND

A wearable device is a portable device that is worn directly on a body or is integrated with a user's clothing and accessory. The wearable device is a hardware device that uses software support, data interaction, and cloud interaction to implement various functions. In the prior art, the following two manners are mainly used to detect whether the wearable device has been worn on the user's body. One is to use a capacitive sensor to perform detection. However, when the capacitive sensor is in use, the capacitive sensor fails when a metal object is close to the capacitive sensor, and a detection success rate is low. The other is to use a visible light sensor to perform detection. However, the visible light sensor cannot identify whether a human body is close to the wearable device or another object is close to the wearable device, and a detection success rate is also low. If it cannot be determined whether the wearable device has been worn on the user's body, the wearable device cannot be used, thereby causing inconvenience to the user.

US 2008/045806 A1 describes methods to sense biometric data, physiological data and security conditions and transmit a signal if conditions are met.

US 2014/275852 A1 describes methods of operating a heart rate monitor of a wearable fitness monitoring device having a plurality of sensors including the heart rate monitor.

US 2012/215115 A1 describes a pulse detector that detects a pulse signal originating from a pulse of a subject

### SUMMARY

The present invention provides a method for detecting a wearable device, and the wearable device. In the method, whether a user has worn the wearable device can be determined quickly and accurately by detecting a value of a distance between the wearable device and the user, and a body feature value of the user.

Herein described is a method for detecting a wearable device, including:
detecting a value of a distance between the wearable device and a user;
determining whether the value of the distance between the wearable device and the user exceeds a preset distance threshold;
if the value of the distance between the wearable device and the user does not exceed the preset distance threshold, detecting a body feature value of the user; and
if the body feature value of the user does not exceed a preset threshold range of the body feature value of the user, determining that the user has worn the wearable device.

Herein described is, after the determining that the user has worn the wearable device, the method further includes:
setting a preset time period; and
detecting the body feature value of the user according to the set preset time period.

Herein described is, the preset time period is set by the user.

Herein described is, the method further includes:
if the value of the distance between the wearable device and the user exceeds the preset distance threshold, determining that the wearable device is not worn by the user.

Herein described is, the method further includes:
if the body feature value of the user exceeds a preset threshold of the body feature value of the user, determining that the wearable device is not worn by the user.

Herein described is,
the body feature value of the user includes one or more of a temperature value, a heart rate value, a blood glucose value, a blood pressure value, or a blood flow velocity value.

Herein described is,
the value of the distance between the wearable device and the user is obtained by using a distance sensor.

Herein described is,
the body feature value of the user is obtained by using a human body feature sensor.

Herein described is a wearable device, including: a distance sensor, a human body feature sensor, and a processor, where
the distance sensor is configured to detect a value of a distance between the wearable device and a user;
the human body feature sensor is configured to detect a body feature value of the user; and
the processor is configured to: determine whether the value of the distance between the wearable device and the user exceeds a preset distance threshold, if the value of the distance between the wearable device and the user does not exceed the preset distance threshold, control the human body feature sensor to detect the body feature value of the user, and if the body feature value of the user does not exceed a preset threshold range of the body feature value of the user, determine that the user has worn the wearable device.

Herein described is, the processor is further configured to:
set a preset time period; and
detect the body feature value of the user according to the set preset time period.

Herein described is, the preset time period is set by the user.

Herein described is, the processor is further configured to:
if the value of the distance between the wearable device and the user exceeds the preset distance threshold, determine that the wearable device is not worn by the user.

Herein described is, the processor is further configured to:
if the body feature value of the user exceeds a preset threshold of the body feature value of the user, determine that the wearable device is not worn by the user.

Herein described is,
the body feature value of the user includes one or more of a temperature value, a heart rate value, a blood glucose value, a blood pressure value, or a blood flow velocity value.

According to the method for detecting a wearable device, and the wearable device of the present invention, a value of a distance between the wearable device and a user is
first detected, then if the value of the distance between the wearable device and the user does not exceed a preset distance threshold, whether a body feature value of the user exceeds a threshold range is detected, and if the body feature value of the user does not exceed the threshold range, that the user has worn the wearable device is determined. That the user has worn the wearable device is determined by combining the value of the distance between the wearable device and the user and the body feature value of the user, so that whether the wearable device is worn on a human body can be identified accurately, and detection accuracy is high.

In addition, in the present invention, the value of the distance between the wearable device and the user is first detected, and the body feature value is not detected at the same time, thereby avoiding that a detection time is prolonged when the value of the distance and the body feature value are detected at the same time. Moreover, if the value of the distance between the wearable device and the user exceeds the preset distance threshold, there is no need to detect the body feature value of the user, thereby optimizing a detection process. Therefore, compared with the prior art, in the embodiments of the present invention, whether the user has worn the wearable device can be determined more quickly and more accurately.

### BRIEF DESCRIPTION OF DRAWINGS

To describe the technical solutions in the embodiments of the present invention or in the prior art more clearly, the following briefly describes the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 is a schematic structural diagram of a smart watch according to an embodiment of the present invention;
FIG. 2 is a schematic structural diagram of smart glasses according to an embodiment of the present invention;
FIG. 3 is a schematic structural diagram of a smart band according to an embodiment of the present invention;
FIG. 4 is a schematic flowchart of a method for detecting a wearable device according to a first embodiment of the present invention;
FIG. 5 is a schematic flowchart of a method for detecting a wearable device according to a second embodiment of the present invention; and
FIG. 6 is a schematic structural diagram of a wearable device according to an embodiment of the present invention.

### DESCRIPTION OF EMBODIMENTS

The following clearly and completely describes the technical solutions in the embodiments of the present invention with reference to the accompanying drawings in the embodiments of the present invention. Apparently, the described embodiments are a part rather than all of the embodiments of the present invention.

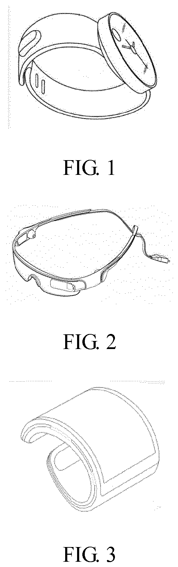

Referring to FIG. 1, FIG. 2, and FIG. 3, during specific implementation, a wearable device described in the embodiments of the present invention may include: a smart watch, a smart band, a smart ring, smart glasses, and the like. The foregoing wearable devices are merely exemplary rather than exhaustive. The wearable device includes but is not limited to the foregoing wearable devices.

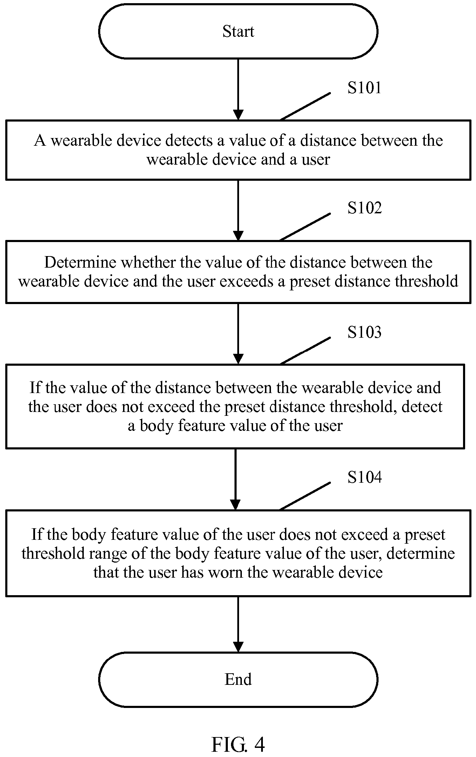

Referring to FIG. 4, FIG. 4 is a schematic flowchart of a method for detecting a wearable device according to a first embodiment of the present invention, the method specifically includes the following steps.

S101. The wearable device detects a value of a distance between the wearable device and a user.

Specifically, the wearable device detects the value of the distance between the wearable device and the user by using a distance sensor, and determines, by using the value of the distance between the wearable device and the user, whether the user wears the wearable device. It should be understood that, the distance sensor in this embodiment of the present invention includes an optical proximity sensor, a linear proximity sensor, an ultrasonic displacement sensor, and the like.

S102. Determine whether the value of the distance between the wearable device and the user exceeds a preset distance threshold.

Specifically, the wearable device obtains the value of the distance between the wearable device and the user, and determines whether the value is less than or equal to the preset distance threshold. It should be understood that, a value range of the preset distance threshold is greater than or equal to 0 cm, and is less than or equal to 2 cm. For example, if the value, obtained by the wearable device, of the distance between the wearable device and the user is 1.5 cm, the value is less than the preset distance threshold. For another example, if the value, obtained by the wearable device, of the distance between the wearable device and the user is 2 cm, the value is equal to the preset distance threshold. For another example, if the value, obtained by the wearable device, of the distance between the wearable device and the user is 2.5 cm, the value is greater than the preset distance threshold.

S103. If the value of the distance between the wearable device and the user does not exceed the preset distance threshold, detect a body feature value of the user.

Specifically, when the value of the distance between the wearable device and the user does not exceed the preset distance threshold, the wearable device obtains the body feature value of the user by using a human body feature sensor. The body feature value of the user includes one or more of a temperature value, a heart rate value, a blood glucose value, a blood pressure value, or a blood flow velocity value. According to the invention,
the wearable device obtains the temperature value of the user by using a temperature sensor on the human body feature sensor, or obtains the heart rate value of the user by using a heart rate sensor on the human body feature sensor, or obtains the blood flow velocity value of the user by using the human body feature sensor. Certainly, in another implementation manner of this embodiment of the present invention, the wearable device may also obtain a body feature value such as the blood glucose value or the blood pressure value by using the human body feature sensor. These body feature values all can reflect a body status of the user, and these body feature values are easy to obtain.

S104. If the body feature value of the user does not exceed a preset threshold range of the body feature value of the user, determine that the user has worn the wearable device.

According to the invention, a preset threshold value range of the temperature value of the user is 36°C to 38°C, and if the temperature value of the user obtained by the wearable device by using the temperature sensor is for example
35°C, the body feature value of the user
exceeds the preset threshold range of the body feature value of the user. For another example, the preset threshold value range of the heart rate value of the according to the invention is 60 beats
to 100 beats per minute, and if the heart rate value of the user obtained by the heart rate sensor on the human body feature sensor is for example 75 beats per minute, the body feature
value of the user does not exceed the preset threshold range of the body feature value of the user. For another example, the
preset threshold of a forearm blood flow velocity
of the user according to the invention
is 6 ml/min/100 g to 10 ml/min/100 g, and when a forearm skin blood flow velocity of the user detected by the wearable device is 8 ml/min/100 g, the body feature value of the user does not exceed the preset threshold range of the body feature value of the user. Similarly, thresholds of the blood pressure value and the blood glucose value may also be set in advance. After collecting the body feature value information, the wearable device compares the body feature value with the preset threshold to determine whether the body feature value exceeds the preset threshold range. According to an actual requirement, the wearable device may collect several body feature values of the user at the same time, or just collect one body feature value of the user.

It should be noted that, when the value of the distance between the wearable device and the user is less than or equal to the preset distance threshold, the body feature value of the user is detected. If the value of the distance between the wearable device and the user exceeds the preset distance threshold, it is directly determined that the wearable device is not worn by the user. Certainly, if the body feature value of the user exceeds the preset threshold of the body feature value of the user, it is determined that the wearable device is not worn by the user.

According to the method for detecting a wearable device, and the wearable device of the present invention, a value of a distance between the wearable device and a user may be first detected, then if the value of the distance between the wearable device and the user does not exceed a preset distance threshold, whether a body feature value of the user exceeds a threshold range is detected, and if the body feature value of the user does not exceed the threshold range, that the user has worn the wearable device is determined. That the user has worn the wearable device is determined by combining the value of the distance between the wearable device and the user and the body feature value of the user, so that whether the wearable device is worn on a human body can be identified accurately, and detection accuracy is high. In addition, in the present invention, the value of the distance between the wearable device and the user is first detected, and the body feature value is not detected at the same time, thereby avoiding that a detection time is prolonged when the value of the distance and the body feature value are detected at the same time. Moreover, if the value of the distance between the wearable device and the user exceeds the preset distance threshold, there is no need to detect the body feature value of the user, thereby optimizing a detection process. Therefore, compared with the prior art, in this embodiment of the present invention, whether the user has worn the wearable device can be determined more quickly and more accurately.

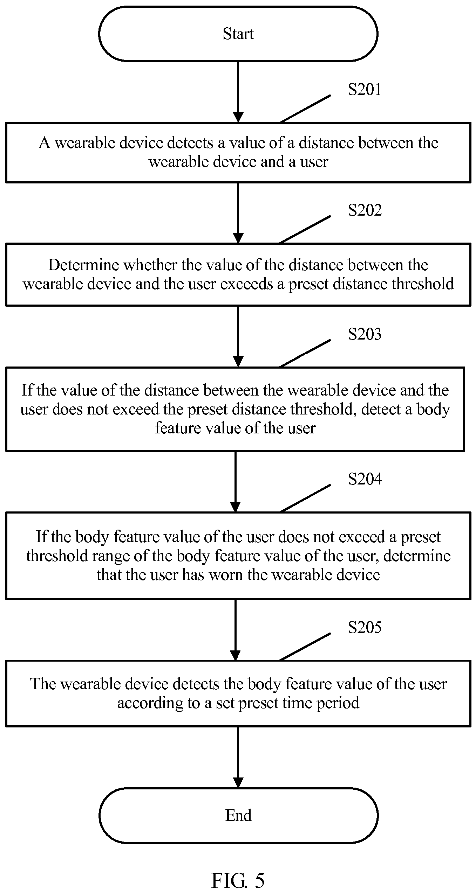

Referring to FIG. 5, FIG. 5 is a schematic flowchart of a method for detecting a wearable device according to a second embodiment of the present invention, the method specifically includes the following steps.

S201. The wearable device detects a value of a distance between the wearable device and a user.

Specifically, the wearable device detects the value of the distance between the wearable device and the user by using a distance sensor or an optical proximity sensor, and determines, by using the value of the distance between the wearable device and the user, whether the user wears the wearable device. It should be understood that, the distance sensor in this embodiment of the present invention includes the optical proximity sensor, a linear proximity sensor, an ultrasonic displacement sensor, and the like.

S202. Determine whether the value of the distance between the wearable device and the user exceeds a preset distance threshold.

Specifically, the wearable device obtains the value of the distance between the wearable device and the user, and determines whether the value is less than or equal to the preset distance threshold. It should be understood that, a value range of the preset distance threshold is greater than or equal to 0 cm, and is less than or equal to 2 cm. For example, if the value, obtained by the wearable device, of the distance between the wearable device and the user is 1.5 cm, the value is less than the preset distance threshold. For another example, if the value, obtained by the wearable device, of the distance between the wearable device and the user is 2 cm, the value is equal to the preset distance threshold. For another example, if the value, obtained by the wearable device, of the distance between the wearable device and the user is 2.5 cm, the value is greater than the preset distance threshold.

S203. If the value of the distance between the wearable device and the user does not exceed the preset distance threshold, detect a body feature value of the user.

Specifically, when the value of the distance between the wearable device and the user does not exceed the preset distance threshold, the wearable device obtains the body feature value of the user by using a human body feature sensor. The body feature value of the user includes one or more of a temperature value, a heart rate value, a blood glucose value, a blood pressure value, or a blood flow velocity value.

According to the invention, the wearable device obtains the temperature of the user by using a temperature sensor on the human body feature sensor, or obtains the heart rate value of the user by using a heart rate sensor on the human body feature sensor, or obtains the blood flow velocity value of the user by using the human body feature sensor. Certainly, in another implementation manner of this embodiment of the present invention, the wearable device may also obtain a body feature value such as the blood glucose value or the blood pressure by using the human body feature sensor. These body feature values all can reflect a body status of the user, and these body feature values are easy to obtain.

S204. If the body feature value of the user does not exceed a preset threshold range of the body feature value of the user, determine that the user has worn the wearable device.

According to the invention, a preset threshold value range of the temperature value of the user is 36°C to 38°C, and if the temperature value of the user obtained by the wearable device by using the temperature sensor is for example 35°C, the body feature value of the user
exceeds the preset threshold range of the body feature value of the user. For another example, the
preset threshold value range of the heart rate value of the user according to the invention is 60 beats
to 100 beats per minute, and if the heart rate value of the user obtained by the heart rate sensor on the human body feature sensor is for example 75 beats per minute, the body feature
value of the user does not exceed the preset threshold range of the body feature value of the user. For another example, the
preset threshold of a forearm blood flow velocity
of the user according to the invention
is 6 ml/min/100 g to 10 ml/min/100 g, and when a forearm skin blood flow velocity of the user detected by the wearable device is 8 ml/min/100 g, the body feature value of the user does not exceed the preset threshold range of the body feature value of the user. Similarly, thresholds of the blood pressure value and the blood glucose value may also be set in advance. After collecting the body feature value information, the wearable device compares the body feature value with the preset threshold to determine whether the body feature value exceeds the preset threshold range. According to an actual requirement, the wearable device may collect several body feature values of the user at the same time, or just collect one body feature value of the user.

It should be noted that, when the value of the distance between the wearable device and the user is less than or equal to the preset distance threshold, the body feature value of the user is detected. If the value of the distance between the wearable device and the user exceeds the preset distance threshold, it is directly determined that the wearable device is not worn by the user. Certainly, if the body feature value of the user exceeds the preset threshold of the body feature value of the user, it is determined that the wearable device is not worn by the user.

S205. The wearable device detects the body feature value of the user according to a set preset time period.

Specifically, after it is determined that the user has worn the wearable device, the user may set the preset time period, and the wearable device detects the body feature value of the user according to the set preset time period. The preset time period includes 5 minutes to 10 minutes. If the wearable device that has been worn by the user is taken off, there is no need to detect the distance between the wearable device and the user again, only the body feature value of the user needs to be detected, there is no need to detect the body feature value of the user in real time, the wearable device just needs to detect the body feature value of the user every 5 minutes to 10 minutes, and whether the user wears the wearable device can be determined quickly and accurately.

According to the method for detecting a wearable device, and the wearable device of the present invention, a value of a distance between the wearable device and a user is
first detected, then if the value of the distance between the wearable device and the user does not exceed a preset distance threshold, whether a body feature value of the user exceeds a threshold range is detected, and if the body feature value of the user does not exceed the threshold range, that the user has worn the wearable device is determined. That the user has worn the wearable device is determined by combining the value of the distance between the wearable device and the user and the body feature value of the user, so that whether the wearable device is worn on a human body can be identified accurately, and detection accuracy is high. In the present invention, the value of the distance between the wearable device and the user is first detected, and the body feature value is not detected at the same time, thereby avoiding that a detection time is prolonged when the value of the distance and the body feature value are detected at the same time. Moreover, if the value of the distance between the wearable device and the user exceeds the preset distance threshold, there is no need to detect the body feature value of the user, thereby optimizing a detection process. Therefore, compared with the prior art, in this embodiment of the present invention, whether the user has worn the wearable device can be determined more quickly and more accurately.

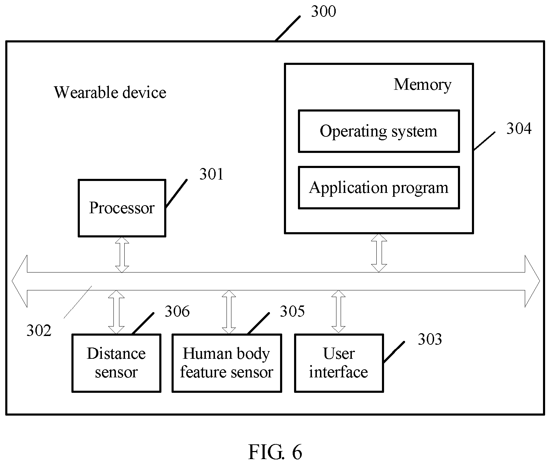

Referring to FIG. 6, FIG. 6 is a schematic structural diagram of a wearable device according to an embodiment of the present invention, the wearable device 300 includes: at least one processor 301, a human body feature sensor 305, and a distance sensor 306, and further includes: the at least one user interface 303, the at least one communications bus 302, and the at least one memory 304.

The processor 301 may include a CPU. The human body feature sensor 305 may include a temperature sensor, a heart rate sensor, a blood glucose sensor, a blood pressure sensor, a blood flow velocity sensor, and the like. The distance sensor 306 may include an optical proximity sensor, a linear proximity sensor, an ultrasonic displacement sensor, and the like. The communications bus 302 is configured to implement connection and communication between these components. The user interface 303 may include a display (Display) and a keyboard (Keyboard). Optionally, the user interface 303 may further include a standard wired interface and a standard wireless interface. The memory 304 may be a high-speed RAM memory, or may be a non-volatile memory (non-volatile memory), for example, at least one disk memory. Optionally, the memory 304 may be at least one storage apparatus far from the foregoing processor 301.

The memory 304 stores a group of program code, and the processor 301 invokes the program code stored in the memory 304 to perform the following operations:
the distance sensor 306 is configured to detect a value of a distance between the wearable device and a user;
the human body feature sensor 305 is configured to detect a body feature value of the user; and
the processor 301 is configured to determine whether the value of the distance between the wearable device and the user exceeds a preset distance threshold, if the value of the distance between the wearable device and the user does not exceed the preset distance threshold, control the human body feature sensor to detect the body feature value of the user, and if the body feature value of the user does not exceed a preset threshold range of the body feature value of the user, determine that the user has worn the wearable device.

Specifically, the wearable device detects the value of the distance between the wearable device and the user by using the distance sensor or the optical proximity sensor, and determines, by using the value of the distance between the wearable device and the user, whether the user wears the wearable device.

The wearable device obtains the value of the distance between the wearable device and the user, and determines whether the value is less than or equal to the preset distance threshold. It should be understood that, a value range of the preset distance threshold is greater than or equal to 0 cm, and is less than or equal to 2 cm. For example, if the value, obtained by the wearable device, of the distance between the wearable device and the user is 1.5 cm, the value is less than the preset distance threshold. For another example, if the value, obtained by the wearable device, of the distance between the wearable device and the user is 2 cm, the value is equal to the preset distance threshold. For another example, if the value, obtained by the wearable device, of the distance between the wearable device and the user is 2.5 cm, the value is greater than the preset distance threshold.

In an optional embodiment, the processor 301 may invoke the program code stored in the memory 304 to further perform the following operations:
set a preset time period; and
detect the body feature value of the user according to the set preset time period.

Specifically, after determining that the user has worn the wearable device, the wearable device detects the body feature value of the user according to the time period preset by the user. The time period preset by the user may include 5 minutes to 10 minutes. If the wearable device that has been worn by the user is taken off, there is no need to detect the distance between the wearable device and the user again, only the body feature value of the user needs to be detected, there is no need to detect the body feature value of the user in real time, the wearable device just needs to detect the body feature value of the user every 5 minutes to 10 minutes, and whether the user wears the wearable device can be determined quickly and accurately.

In an optional embodiment, the processor 301 may invoke the program code stored in the memory 304 to further perform the following operation:
the preset time period is set by the user.

In an optional embodiment, the processor 301 may invoke the program code stored in the memory 304 to further perform the following operation:
if the value of the distance between the wearable device and the user exceeds the preset distance threshold, determine that the wearable device is not worn by the user.

In an optional embodiment, the processor 301 may invoke the program code stored in the memory 304 to further perform the following operation:
if the body feature value of the user exceeds a preset threshold of the body feature value of the user, determine that the wearable device is not worn by the user.

In an optional embodiment, the processor 301 may invoke the program code stored in the memory 304 to further perform the following operation:
the body feature value of the user includes one or more of a temperature value, a heart rate value, a blood glucose value, a blood pressure value, or a blood flow velocity value.

In an optional embodiment, the processor 301 may invoke the program code stored in the memory 304 to further perform the following operation:
the value of the distance between the wearable device and the user is obtained by using the distance sensor.

In an optional embodiment, the processor 301 may invoke the program code stored in the memory 304 to further perform the following operation:
the body feature value of the user is obtained by using the human body feature sensor.

Specifically, when the value of the distance between the wearable device and the user does not exceed the preset distance threshold, the wearable device obtains the body feature value of the user by using the human body feature sensor. The body feature value of the user includes one or more of a temperature value, a heart rate value, a blood glucose value, a blood pressure value, or a blood flow velocity value. According to the invention,
the wearable device obtains the temperature value of the user by using the temperature sensor on the human body feature sensor, or obtains the heart rate value of the user by using the heart rate sensor on the human body feature sensor, or obtains the blood flow velocity value of the user by using the human body feature sensor. Certainly, in another implementation manner of this embodiment of the present invention, the wearable device may also obtain a body feature value such as the blood glucose value or the blood pressure value by using the human body feature sensor. These body feature values all can reflect a body status of the user, and these body feature values are easy to obtain.

According to the invention, the preset threshold value range of the temperature value of the user is 36°C to 38°C, and if the temperature value of the user obtained by the wearable device by using the temperature sensor is 35°C, the body feature value of the user exceeds the preset threshold range of the body feature value of the user. For another example, the
preset threshold value range of the heart rate value of the user according to the invention
is 60 beats to 100 beats per minute, and if the heart rate value of the user obtained by the heart rate sensor on the human body feature sensor is 75 beats per minute, the body feature value of the user does not exceed the preset threshold range of the body feature value of the user. For another example, the
preset threshold of a
forearm blood flow velocity of the user according to the invention is 6 ml/min/100 g to 10 ml/min/100 g, and
when a forearm skin blood flow velocity of the user detected by the wearable device is 8 ml/min/100 g, the body feature value of the user does not exceed the threshold range of the body feature value of the user. Similarly, thresholds of the blood pressure value and the blood glucose value may also be set in advance. After collecting the body feature value information, the wearable device compares the body feature value with the preset threshold to determine whether the body feature value exceeds the preset threshold range. According to an actual requirement, the wearable device may collect several body feature values of the user at the same time, or just collect one body feature value of the user.

It should be noted that, when the value of the distance between the wearable device and the user is less than or equal to the preset distance threshold, the body feature value of the user is detected. If the value of the distance between the wearable device and the user exceeds the preset distance threshold, it is directly determined that the wearable device is not worn by the user. Certainly, if the body feature value of the user exceeds the preset threshold of the body feature value of the user, it is determined that the wearable device is not worn by the user.

According to the method for detecting a wearable device, and the wearable device in this embodiment of the present invention, a value of a distance between the wearable device and a user is
first detected, then if the value of the distance
between the wearable device and the user does not exceed a preset distance threshold, whether a body feature value of the user exceeds a threshold range is detected, and if the body feature value of the user does not exceed the threshold range, that the user has worn the wearable device is determined. That the user has worn the wearable device is determined by combining the value of the distance between the wearable device and the user and the body feature value of the user, so that whether the wearable device is worn on a human body can be identified accurately, and detection accuracy is high. In addition, in this embodiment of the present invention, the value of the distance between the wearable device and the user is first detected, and the body feature value is not detected at the same time, thereby avoiding that a detection time is prolonged when the value of the distance and the body feature value are detected at the same time. Moreover, if the value of the distance between the wearable device and the user exceeds the preset distance threshold, there is no need to detect the body feature value of the user, thereby optimizing a detection process. Therefore, compared with the prior art, in this embodiment of the present invention, whether the user has worn the wearable device can be determined more quickly and more accurately.

It should be understood that, the first execution unit and the second execution unit do not indicate a sequenced relationship, but are to differentiate different execution units. Similarly, a first detection unit and a second detection unit are to indicate different detection units, and details are not described herein.

A person of ordinary skill in the art may understand that, each aspect of the present invention or a possible implementation manner of each aspect may be specifically implemented as a system, a method, or a computer program product. In addition, some aspects
of the present invention or the possible implementation manner
of some aspects
may take a form of a computer program product, where the computer program product refers to computer-readable program code stored in a computer-readable medium.

The computer-readable medium may be a computer-readable data medium or a computer-readable storage medium. The computer-readable storage medium includes but is not limited to an electronic, magnetic, optical, electromagnetic, infrared, or semi-conductive system, device, or apparatus, or any appropriate combination thereof, such as a random access memory (RAM), a read-only memory (ROM), an erasable programmable read only memory (EPROM or flash memory), an optical fiber, and a compact disc read only memory (CD-ROM).

A processor in a computer reads computer-readable program code stored in a computer-readable medium, so that the processor can perform a function and an action specified in each step or a combination of steps in a flowchart; an apparatus is generated to implement a function and an action specified in each block or a combination of blocks in a block diagram.

All computer-readable program code may be executed on a user computer, or some may be executed on a user computer as a standalone software package, or some may be executed on a local computer of a user while some is executed on a remote computer, or all the code may be executed on a remote computer or a server.

Obviously, a person skilled in the art can make various modifications and variations to the present invention without departing from scope of the present invention. The present invention is intended to cover these modifications and variations provided that they fall within the scope of protection defined by the following claims.

## Claims

1. A method for detecting a wearable device, comprising:
detecting (S101), by a distance sensor (306), a value of a distance between the wearable device and a user;
determining (S102), by a processor (301), whether the value of the distance between the wearable device and the user exceeds a preset distance threshold;
if the value of the distance between the wearable device and the user does not exceed the preset distance threshold, detecting (S103), by a human body feature sensor (305), a body feature value of the user; and
if the body feature value of the user does not exceed a preset threshold range of the body feature value of the user, determining (S104) that the user has worn the wearable device;
wherein the preset distance threshold is greater than or equal to 0 cm and less than or equal to 2 cm; and
wherein the body feature value of the user comprises one or more of:
- a temperature value, and the preset threshold value range of the temperature value of the user is 36°C to 38°C;
- a heart rate value, and the preset threshold value range of the heart rate value of the user is 60 beats to 100 beats per minute;
- blood flow velocity value, and the preset threshold value range of a forearm blood flow velocity of the user is 6 ml/min/100 g to 10 ml/min/100 g.

2. The method according to claim 1, wherein after the determining that the user has worn the wearable device, the method further comprises:
setting a preset time period; and
detecting the body feature value of the user according to the preset time period.

3. The method according to claim 2, wherein the preset time period is set by the user.

4. The method according to claim 1, wherein the method further comprises:
if the value of the distance between the wearable device and the user exceeds the preset distance threshold, determining that the wearable device is not worn by the user.

5. The method according to claim 1, wherein the method further comprises:
if the body feature value of the user exceeds a preset threshold of the body feature value of the user, determining that the wearable device is not worn by the user.

6. The method according to any one of claims 1 to 5, wherein:
the body feature value of the user comprises: one or more of a temperature value, a heart rate value, a blood glucose value, a blood pressure value, or a blood flow velocity value.

7. A wearable device, comprising: a distance sensor (306), a human body feature sensor (305), and a processor (301), wherein:
the distance sensor is configured to detect a value of a distance between the wearable device and a user;
the human body feature sensor is configured to detect a body feature value of the user; and
the processor is configured to: determine whether the value of the distance between the wearable device and the user exceeds a preset distance threshold, if the value of the distance between the wearable device and the user does not exceed the preset distance threshold, control the human body feature sensor to detect the body feature value of the user, and if the body feature value of the user does not exceed a preset threshold range of the body feature value of the user, determine that the user has worn the wearable device;
wherein the preset distance threshold is greater than or equal to 0 cm and less than or equal to 2 cm; and
wherein the body feature value of the user comprises one or more of:
- a temperature value, and the preset threshold value range of the temperature value of the user is 36°C to 38°C;
- a heart rate value, and the preset threshold value range of the heart rate value of the user is 60 beats to 100 beats per minute;
- blood flow velocity value, and the preset threshold value range of a forearm blood flow velocity of the user is 6 ml/min/100 g to 10 ml/min/100 g.

8. The wearable device according to claim 7,
wherein the processor is further configured to:
set a preset time period; and
detect the body feature value of the user according to the preset time period.

9. The wearable device according to claim 8, wherein the preset time period is set by the user.

10. The wearable device according to claim 7,
wherein the processor is further configured to:
if the value of the distance between the wearable device and the user exceeds the preset distance threshold, determine that the wearable device is not worn by the user.

11. The wearable device according to claim 7,
wherein the processor is further configured to:
if the body feature value of the user exceeds a preset threshold of the body feature value of the user, determine that the wearable device is not worn by the user.

12. The wearable device according to any one of claims 7 to 11,
wherein:
the body feature value of the user comprises: one or more of a temperature value, a heart rate value, a blood glucose value, a blood pressure value, or a blood flow velocity value.

## Patentansprüche

1. Verfahren zum Erkennen einer am Körper tragbaren Vorrichtung, umfassend:
Erkennen (S101) eines Werts eines Abstands zwischen der am Körper tragbaren Vorrichtung und einem Benutzer durch einen Abstandssensor (306);
Bestimmen (S102), ob der Wert des Abstands zwischen der am Körper tragbaren Vorrichtung und dem Benutzer eine voreingestellte Abstandsschwelle übersteigt, durch einen Prozessor (301);
wenn der Wert des Abstands zwischen der am Körper tragbaren Vorrichtung und dem Benutzer die voreingestellte Abstandsschwelle nicht übersteigt, Erkennen (S103) eines Körpermerkmalwerts des Benutzers durch einen Menschenkörpermerkmalsensor (305);
und
wenn der Körpermerkmalwert des Benutzers einen voreingestellten Schwellenbereich des Körpermerkmalwerts des Benutzers nicht übersteigt, Bestimmen (S104), dass der Benutzer die am Körper tragbare Vorrichtung getragen hat;
wobei die voreingestellte Abstandsschwelle größer oder gleich 0 cm und kleiner oder gleich 2 cm ist; und
wobei der Körpermerkmalwert des Benutzers einen oder mehrere der Folgenden umfasst:
- einen Temperaturwert, und der voreingestellte Schwellenwertbereich des Temperaturwerts des Benutzers ist 36 °C bis 38 °C;
- einen Herzfrequenzwert, und der voreingestellte Schwellenwertbereich des Herzfrequenzwerts des Benutzers ist 60 Schläge bis 100 Schläge pro Minute;
- Blutflussgeschwindigkeitswert, und der voreingestellte Schwellenwertbereich einer Unterarm-Blutflussgeschwindigkeit des Benutzers ist 6 ml/min/100 g bis 10 ml/min/100 g.

2. Verfahren nach Anspruch 1, wobei das Verfahren nach dem Bestimmen, dass der Benutzer die am Körper tragbare Vorrichtung getragen hat, ferner Folgendes umfasst:
Setzen eines voreingestellten Zeitraums; und
Erkennen des Körpermerkmalwerts des Benutzers gemäß dem voreingestellten Zeitraum.

3. Verfahren nach Anspruch 2, wobei der voreingestellte Zeitraum durch den Benutzer gesetzt wird.

4. Verfahren nach Anspruch 1, wobei das Verfahren ferner Folgendes umfasst:
wenn der Wert des Abstands zwischen der am Körper tragbaren Vorrichtung und dem Benutzer die voreingestellte Abstandsschwelle übersteigt, Bestimmen, dass die am Körper tragbare Vorrichtung nicht vom Benutzer getragen wird.

5. Verfahren nach Anspruch 1, wobei das Verfahren ferner Folgendes umfasst:
wenn der Körpermerkmalwert des Benutzers eine voreingestellte Schwelle des Körpermerkmalwerts des Benutzers übersteigt, Bestimmen, dass die am Körper tragbare Vorrichtung nicht vom Benutzer getragen wird.

6. Verfahren nach einem der Ansprüche 1 bis 5, wobei
der Körpermerkmalwert des Benutzers einen oder mehrere eines Temperaturwerts, eines Herzfrequenzwerts, eines Blutglucosewerts, eines Blutdruckwerts oder eines Blutflussgeschwindigkeitwerts umfasst.

7. Am Körper tragbare Vorrichtung, die einen Abstandssensor (306), einen Menschenkörpermerkmalsensor (305) und einen Prozessor (301) umfasst, wobei der Abstandssensor ausgelegt ist zum Erkennen eines Werts eines Abstands zwischen der am Körper tragbaren Vorrichtung und einem Benutzer;
der Menschenkörpermerkmalsensor ausgelegt ist zum Erkennen eines Körpermerkmalwerts des Benutzers; und
der Prozessor für Folgendes ausgelegt ist: Bestimmen, ob der Wert des Abstands zwischen der am Körper tragbaren Vorrichtung und dem Benutzer eine voreingestellte Abstandsschwelle übersteigt, wenn der Wert des Abstands zwischen der am Körper tragbaren Vorrichtung und dem Benutzer die voreingestellte Abstandsschwelle nicht übersteigt, Steuern des Menschenkörpermerkmalsensors, den Körpermerkmalwert des Benutzers zu erkennen, und wenn der Körpermerkmalwert des Benutzers einen voreingestellten Schwellenbereich des Körpermerkmalwerts des Benutzers nicht übersteigt, Bestimmen, dass der Benutzer die am Körper tragbare Vorrichtung getragen hat;
wobei die voreingestellte Abstandsschwelle größer oder gleich 0 cm und kleiner oder gleich 2 cm ist; und
wobei der Körpermerkmalwert des Benutzers einen oder mehrere der Folgenden umfasst:
- einen Temperaturwert, und der voreingestellte Schwellenwertbereich des Temperaturwerts des Benutzers ist 36 °C bis 38 °C;
- einen Herzfrequenzwert, und der voreingestellte Schwellenwertbereich des Herzfrequenzwerts des Benutzers ist 60 Schläge bis 100 Schläge pro Minute;
- Blutflussgeschwindigkeitswert, und der voreingestellte Schwellenwertbereich einer Unterarm-Blutflussgeschwindigkeit des Benutzers ist 6 ml/min/100 g bis 10 ml/min/100 g.

8. Am Körper tragbare Vorrichtung nach Anspruch 7, wobei der Prozessor ferner ausgelegt ist zum
Setzen eines voreingestellten Zeitraums; und
Erkennen des Körpermerkmalwerts des Benutzers gemäß dem voreingestellten Zeitraum.

9. Am Körper tragbare Vorrichtung nach Anspruch 8, wobei der voreingestellte Zeitraum durch den Benutzer gesetzt wird.

10. Am Körper tragbare Vorrichtung nach Anspruch 7, wobei der Prozessor ferner für Folgendes ausgelegt ist:
wenn der Wert des Abstands zwischen der am Körper tragbaren Vorrichtung und dem Benutzer die voreingestellte Abstandsschwelle übersteigt, Bestimmen, dass die am Körper tragbare Vorrichtung nicht vom Benutzer getragen wird.

11. Am Körper tragbare Vorrichtung nach Anspruch 7, der Prozessor ferner für Folgendes ausgelegt ist:
wenn der Körpermerkmalwert des Benutzers eine voreingestellte Schwelle des Körpermerkmalwerts des Benutzers übersteigt, Bestimmen, dass die am Körper tragbare Vorrichtung nicht vom Benutzer getragen wird.

12. Am Körper tragbare Vorrichtung nach einem der Ansprüche 7 bis 11, wobei der Körpermerkmalwert des Benutzers einen oder mehrere eines Temperaturwerts, eines Herzfrequenzwerts, eines Blutglucosewerts, eines Blutdruckwerts oder eines Blutflussgeschwindigkeitwerts umfasst.

## Revendications

1. Procédé de détection d'un dispositif vestimentaire, comprenant :
la détection (S101), par un capteur de distance (306), d'une valeur d'une distance entre le dispositif vestimentaire et un utilisateur ;
la détermination (S102), par un processeur (301), si la valeur de la distance entre le dispositif vestimentaire et l'utilisateur dépasse un seuil de distance prédéfini ;
si la valeur de la distance entre le dispositif vestimentaire et l'utilisateur ne dépasse pas le seuil de distance prédéfini, la détection (S103), par un capteur de caractéristique corporelle humaine (305), d'une valeur de caractéristique corporelle de l'utilisateur ; et
si la valeur de caractéristique corporelle de l'utilisateur ne dépasse pas une plage de seuils prédéfinie de la valeur de caractéristique corporelle de l'utilisateur, la détermination (S104) que l'utilisateur a porté le dispositif vestimentaire ;
le seuil de distance prédéfini étant supérieur ou égal à 0 cm et inférieur ou égal à 2 cm ; et
la valeur de caractéristique corporelle de l'utilisateur comprenant une ou plusieurs des valeurs suivantes :
- une valeur de température, et la plage de valeurs seuils prédéfinies de la valeur de température de l'utilisateur étant comprise entre 36 °C et 38 °C ;
- une valeur de fréquence cardiaque, et la plage de valeurs seuils prédéfinies de la valeur de fréquence cardiaque de l'utilisateur étant comprise entre 60 et 100 battements par minute ;
- la valeur de vitesse du flux sanguin, et la plage de valeurs seuils prédéfinies d'une vitesse du flux sanguin d'un avant-bras de l'utilisateur étant comprise entre 6 ml/min/100 g et 10 ml/min/100 g.

2. Procédé selon la revendication 1, après la détermination que l'utilisateur a porté le dispositif vestimentaire, le procédé comprenant en outre :
la définition d'une période de temps prédéfinie, et
la détection de la valeur de caractéristique corporelle de l'utilisateur en fonction de la période de temps prédéfinie.

3. Procédé selon la revendication 2, la période de temps prédéfinie étant définie par l'utilisateur.

4. Procédé selon la revendication 1, le procédé comprenant en outre :
si la valeur de la distance entre le dispositif vestimentaire et l'utilisateur dépasse le seuil de distance prédéfini, la détermination que le dispositif vestimentaire n'est pas porté par l'utilisateur.

5. Procédé selon la revendication 1, le procédé comprenant en outre :
si la valeur de caractéristique corporelle de l'utilisateur dépasse un seuil prédéfini de la valeur de caractéristique corporelle de l'utilisateur, la détermination que le dispositif vestimentaire n'est pas porté par l'utilisateur.

6. Procédé selon l'une quelconque des revendications 1 à 5,
la valeur de caractéristique corporelle de l'utilisateur comprenant : une ou plusieurs valeurs parmi une valeur de température, une valeur de fréquence cardiaque, une valeur de glycémie, une valeur de pression sanguine ou une valeur de vitesse du flux sanguin.

7. Dispositif vestimentaire, comprenant : un capteur de distance (306), un capteur de caractéristique corporelle humaine (305), et un processeur (301),
le capteur de distance étant configuré pour détecter une valeur d'une distance entre le dispositif vestimentaire et un utilisateur ;
le capteur de caractéristique corporelle humaine étant configuré pour détecter une valeur de caractéristique corporelle de l'utilisateur ; et
le processeur étant configuré pour : déterminer si la valeur de la distance entre le dispositif vestimentaire et l'utilisateur dépasse un seuil de distance prédéfini, si la valeur de la distance entre le dispositif vestimentaire et l'utilisateur ne dépasse pas le seuil de distance prédéfini, commander le capteur de caractéristique corporelle humaine pour détecter la valeur de caractéristique corporelle de l'utilisateur, et si la valeur de caractéristique corporelle de l'utilisateur ne dépasse pas une plage de seuils prédéfinie de la valeur de caractéristique corporelle de l'utilisateur, déterminer que l'utilisateur a porté le dispositif vestimentaire ;
le seuil de distance prédéfini étant supérieur ou égal à 0 cm et inférieur ou égal à 2 cm ; et la valeur de caractéristique corporelle de l'utilisateur comprenant un ou plusieurs des éléments suivants :
- une valeur de température, et la plage de valeurs seuils prédéfinies de la valeur de température de l'utilisateur étant comprise entre 36 °C et 38 °C ;
- une valeur de fréquence cardiaque, et la plage de valeurs seuils prédéfinies de la valeur de fréquence cardiaque de l'utilisateur étant comprise entre 60 et 100 battements par minute ;
- la valeur de vitesse du flux sanguin, et la plage de valeurs seuils prédéfinies d'une vitesse du flux sanguin d'un avant-bras de l'utilisateur étant comprise entre 6 ml/min/100 g et 10 ml/min/100 g.

8. Dispositif vestimentaire selon la revendication 7,
le processeur étant en outre configuré pour :
définir une période de temps prédéfinie ; et
détecter la valeur de caractéristique corporelle de l'utilisateur en fonction de la période de temps prédéfinie.

9. Dispositif vestimentaire selon la revendication 8,
la période de temps prédéfinie étant définie par l'utilisateur.

10. Dispositif vestimentaire selon la revendication 7,
le processeur étant en outre configuré pour :
si la valeur de la distance entre le dispositif vestimentaire et l'utilisateur dépasse le seuil de distance prédéfini, déterminer que le dispositif vestimentaire n'est pas porté par l'utilisateur.

11. Dispositif vestimentaire selon la revendication 7,
le processeur étant en outre configuré pour :
si la valeur de caractéristique corporelle de l'utilisateur dépasse un seuil prédéfini de la valeur de caractéristique corporelle de l'utilisateur, déterminer que le dispositif vestimentaire n'est pas porté par l'utilisateur.

12. Dispositif vestimentaire selon l'une quelconque des revendications 7 à 11,
la valeur de caractéristique corporelle de l'utilisateur comprenant : une ou plusieurs valeurs parmi une valeur de température, une valeur de fréquence cardiaque, une valeur de glycémie, une valeur de pression sanguine ou une valeur de vitesse du flux sanguin.
